# EUROPEAN PATENT SPECIFICATION

(11) **EP 1 549 714 B1**
(45) Date of publication and mention of the grant of the patent: **29.10.2008**
(21) Application number: 03767501.4
(22) Date of filing: 02.10.2003
(51) Int. Cl.: C09C 1/64, C09C 1/62, C09C 1/00, C09C 3/08, C09D 5/36, C09B 69/00, C09B 63/00, C09B 57/00

(54) **METHOD OF COLOURING CARRIER MATERIALS**
VERFAHREN ZUM EINFÄRBEN VON TRÄGERMATERIALIEN
PROCEDE DE COLORATION DE MATIERES DE SUPPORT

(30) Priority: 09.10.2002 EP 02405868
(43) Date of publication of application: 06.07.2005
(73) Proprietor: Ciba Holding Inc., 4057 Basel (CH)
(72) Inventor: MÜLLER, Martin, 79539 Lörrach (DE); BAUER, Didier, F-68680 Kembs (FR)
(86) International application number: PCT/EP2003/010968
(87) International publication number: WO 2004/033563

(56) References cited:
- EP-A- 1 028 146
- WO-A-02/064680
- CH-A- 208 955
- DE-A1- 4 134 600
- DE-A1- 19 618 566
- DE-A1- 19 951 871
- GB-A- 614 407
- US-A- 2 459 771
- US-A- 5 468 289

## Description

The present invention relates to a method of producing coloured carrier materials (substrates), especially "effect pigments", and to the coloured substrates obtained by such a method and also to the use thereof. Using the methods according to the invention, it is possible to obtain colorations and coloured substrates that have surprisingly good light-fastness properties.

Colour pigments and effect pigments are currently enjoying increasing popularity and are a decorative constituent of buildings or means of transport or are used for the optical enhancement of articles of practical use or artistic works. Also, besides the known typical effects, such as, for example, a metallic sheen in copper, bronze, silver or gold hues, pearlescent effects and interference effects, there is, especially, a growing interest in additionally imparting a brightly coloured appearance to such pigments.

Various approaches to satisfying that interest have been taken.

EP-A-810 270 describes a method of colouring aluminium, wherein a solution comprising an acid or an ammonium salt thereof is mixed with an aluminium pigment, whereupon the acid or its ammonium salt is adsorbed onto the surface of the aluminium pigment, and the aluminium pigment obtained is then added to a dispersion of a colour pigment in a non-polar solvent.

In accordance with DE-A-4 225 357, organic pigments and dyes which are soluble in acids without undergoing decomposition are, by means of neutralisation of a dispersion thereof which also comprises substrate particles, precipitated onto the substrate particles.

DE-A-4 225 031 describes a method of producing coloured gloss pigments, wherein substrate particles are dispersed in a solution of the pigment and the pigment is precipitated directly onto the surface of the substrate particles by adding a solvent in which the pigment is insoluble. In the examples, the solvents used are, for the most part, strong acids such as sulfuric acid, polyphosphoric acid or trifluoroacetic acid.

It is furthermore known from DE-A-4 009 567 to deposit colorants such as phthalocyanine and metal phthalocyanine pigments onto a silicate substrate from concentrated sulfuric acid by adding water. Finally, it is also known from DE-A-3 536 168 for vat dyes, in particular 4-methyl-6-chloro-6'-methoxythioindigo, present in the leuco form of the dye in an alkaline solution, to be precipitated from that solution onto the substrate whilst being oxidised to the coloured form of the dye.

US-A-5 718 753 describes coloured metallic pigments, for example aluminium flakes, that are encapsulated in a continuous homogeneous layer of organic pigment particles. The metallic pigments are produced by means of PVD (Physical Vapour Deposition) of the organic pigments onto the aluminium flakes.

US-A-5 156 678 describes the colouring of effect pigments with metal-free phthalocyanine dyes or with phthalocyanine dyes. The dye is dissolved in sulfuric acid and deposited onto the effect pigment by adding water.

The use of adhesion promoters such as resin binder systems (EP-A-403 432), anionic polymers and chromium salts (US-A-4 755 229, US-A-5 931 996) or aluminium hydroxide (DE-A-2 429 762) usually results in dulling of the pigment.

In those methods, the low pigment-substrate affinity, the weak pigment character of the precipitated products and the lack of universal applicability are disadvantageous.

It has now been found, surprisingly, that the adhesion to various substrates and the light-fastness properties can be greatly increased when the substrates are coloured as described hereinbelow.

The present invention accordingly relates to a method of producing coloured carrier particles, which comprises
a) dispersing a pigment in an aqueous solution, preferably water,
b) adding soda waterglass,
c) precipitating SiO₂ and the pigment onto the carrier particles by lowering the pH value, and
also to the carrier particles obtainable in accordance with the method.

The method is carried out in principle as described hereinbelow for the colorant. Where appropriate, in Step b) the pH can be adjusted to a value from 7 to 9 by adding a base to the pigment/soda waterglass dispersion, and the pigment and SiO₂ can be precipitated onto the carrier particles by lowering the pH to a value less than 7. Any desired base can be used in the method. Preference is given to alkali metal hydroxides, especially sodium hydroxide. Lowering of the pH is carried out by adding acid, it being possible in principle to use any acid. Preference is given to hydrochloric acid. The pigments are generally selected from 1-aminoanthraquinone, anthraquinone, anthrapyrimidine, azo, azomethine, benzodifuranone, quinacridone, quinacridone quinone, quinophthalone, diketopyrrolopyrrole, dioxazine, flavanthrone, indanthrone, indigo, isoindoline, isoindolinone, isoviolanthrone, perinone, perylene, phthalocyanine, pyranthrone and thioindigo pigments.

SiO₂ can also be precipitated in analogy to a method described in DE-A-195 01 307, by producing the silicon oxide layer by means of a sol-gel process by controlled hydrolysis of one or more metal acid esters in the presence of one or more of the colorants according to the invention and, optionally, an organic solvent and, optionally, a basic catalyst.

Suitable basic catalysts are, for example, amines, such as triethylamine, ethylenediamine, tributylamine, dimethylethanolamine and methoxypropylamine.

The organic solvent is a water-miscible organic solvent, such as a C₁₋₄alkohol, especially isopropanol.

Suitable metal acid esters are selected from alkyl- and aryl-alcoholates, carboxylates, alkyl alcoholates substituted by carboxyl radicals or alkyl radicals or aryl radicals, and carboxylates of silicon. Preference is given to the use of tetraethyl orthosilicate. Furthermore, acetylacetonates and acetoacetylacetonates of the below-mentioned metals may be used.

The method according to the invention is in principle suitable for colouring any carrier material (substrate). The sole requirement is that the carrier particles should be stable in the solvent without undergoing decomposition. Examples of carrier particles are cellulose (wood), keratin (hair) and anodised aluminium.

The carrier particles are preferably selected from metallic, metal oxide, non-metallic, and (non-metal) oxide carrier particles, especially effect pigments, polymeric compounds and combinations thereof and organic or inorganic pigments.

In general 15 to 100 parts by weight of colorant are used per 100 parts by weight of carrier particles. If the aluminium flakes or flakes on the basis of SiO₂ substrates, such as Iriodin® pigments are used as carrier particles, in particular 20 to 80 parts by weight of colorant are used per 100 parts by weight of carrier particles.

Preference is given to flake-like substrates, the particles of which generally have a length of from 2 µm to 5 mm, a width of from 2 µm to 2 mm and a thickness of from 20 nm to 1.5 µm and a ratio of length to thickness of at least 2:1. It is presently preferred that the diameter of the flakes be in a preferred range of about 2 to 60 µm with a more preferred range of about 5-40 µm.

Metallic effect pigments (metal effect pigments) generally are flake-like particles of aluminium, copper, zinc, gold bronze, titanium (EP-A-796 688), zirconium, tin, iron (EP-A-673 980) or steel or pigments of alloys of the afore-mentioned metals. However, particle shapes other than those of metal flakes are known, such as those of spherical zinc dust or dendritic copper powder. Preference is given to metal flakes, such as aluminium flakes, copper/tin flakes, copper flakes and copper/zinc flakes. Special preference is given to aluminium flakes and coloured aluminium flakes, such as Silberline® (Silberline Inc.), Aloxal 3010 and Metallux 2154 (Carl Eckart GmbH & Co.).

Examples of metal oxide effect pigments are effect pigments based on aluminium oxide (Al₂O₃), zinc oxide (ZnO), zirconium oxide (ZrO₂), titanium dioxide (TiO₂), indium oxide (In₂O₃), indium tin oxide (ITO), tantalum pentoxide (Ta₂O₅), cerium oxide (CeO₂) yttrium oxide (Y₂O₃), europium oxide (Eu₂O₃), iron oxides, such as Fe₃O₄ and Fe₂O₃, hafnium oxide (HfO₂), lanthanum oxide (La₂O₃), magnesium oxide (MgO), neodymium oxide (Nd₂O₃), praseodymium oxide (Pr₆O₁₁), samarium oxide (Sm₂O₃), antimony trioxide (Sb₂O₃), tin oxide (SnO₂), tungsten trioxide (WO₃) or combinations thereof, or effect pigments comprising layers of the afore-mentioned metal oxides.

Examples of (non-metal) oxide effect pigments are effect pigments based on SiOₓ wherein 0.03 ≤ x ≤ 0.95 or SiO_{y} wherein 0.95 < y ≤ 2.0, especially silicon monoxide (SiO), silicon dioxide (SiO₂) or selenium trioxide (Se₂O₃), or effect pigments comprising layers of the afore-mentioned materials such as, for example, SiO₂ flakes.

Examples of non-metallic effect pigments are effect pigments based on BC, BN, SiC or Si₃N₄, or effect pigments comprising layers of the afore-mentioned materials (see, for example, EP 02 405 649.1).

Further examples are effect pigments based on metal fluorides, such as magnesium fluoride (MgF₂), aluminium fluoride (AlF₃), cerium fluoride (CeF₃), lanthanum fluoride (LaF₃), zinc sulfide (ZnS), carbon, hafnium nitride (HfN), hafnium carbide (HfC), sodium aluminium fluorides (for example, Na₃AlF₆ or Na₅Al₃F₁₄), neodymium fluoride (NdF₃), samarium fluoride (SmF₃), barium fluoride (BaF₂), calcium fluoride (CaF₂), lithium fluoride (LiF) or combinations thereof, and effect pigments comprising layers of the afore-mentioned materials, and effect pigments comprising organic monomers and polymers, including dienes or alkenes, such as acrylates (for example, methacrylates), perfluoroalkenes, polytetrafluoroethylene (Teflon), fluorinated ethylenepropylene (FEP) and combinations thereof.

In the case of multi-layer pigments, the afore-mentioned materials, especially metals, metal oxides and (non-metal) oxides, may be included in the layered structure, wherein layers comprising Al, SiOₓ (0.03 ≤ x ≤ 0.95), SiO_{y} (0.95 < y ≤ 2.0), TiO₂ and SiO₂ (see, for example, EP-A-803 549, WO2003/076520 A2, WO04/020530 A1 and WO03/68868 A2) are preferred and colour-imparting oxides of metals such as Fe, Co, Mn, Sn, Cr, Ni, Cu are also possible.

Examples of preferred effect pigments are pearlescent pigments based on the natural mineral mica or on mica already encapsulated in a thin layer of metal oxide, for example titanium dioxide and/or iron oxide, such as Iriodin® 9103 (Merck), Xirallic® effect pigments (Merck) based on aluminium oxide (Al₂O₃) flakes or on Al₂O₃ flakes already coated with metal oxides, and Colorstream^{®} pigments based on synthetically produced SiO₂ (silicon dioxide) flakes already covered with metal oxides.

The method comprises the colouring of substrates using colorants , which is described in detail hereinbelow.

The method comprises
a) dispersing the carrier particles in a solution of a colorant, adding the carrier particles to a solution of a colorant or adding a colorant to a dispersion of the carrier particles,
b) precipitating the colorant onto the carrier particles.

In a preferred embodiment, the present invention relates to a method of producing coloured carrier particles, which comprises
a1) dissolving a colorant in a solvent,
a2) adding the carrier particles to the solution prepared in Step a1),
b) precipitating the colorant onto the carrier particles.

In the method, either there is used a colorant which is soluble in an alkaline medium and which, in Step b), is precipitated onto the substrate by adding acid and/or metal salts, or there is used a colorant which is soluble in a weakly acid or neutral medium and which, in Step b), is precipitated onto the substrate by adding acid and/or metal salts.

The method according to the invention makes use of the pH-dependent solubility or differing solubility of the salts of the colorants, there being achieved a high affinity with the substrate and, where applicable, π-π stabilisation of the deposited colorants by means of suitable functional groups and modifications.

In general, a colorant is used which is soluble in an alkaline medium and which, in Step b), is precipitated onto the substrate by adding acid and/or ammonium or metal salts, such as an alkali metal salt, alkaline earth metal salt, aluminium salt or transition metal salt, especially NH₄⁺, ⁺NR³¹R³²R³³R³⁴, wherein R³¹, R³², R³³ and R³⁴ are as defined hereinbelow, Na, K, Mg, Ca, Sr, Ba, Mn, Cu, Ni, Cd, Co, Cr, Zn, Al or Fe, or a colorant is used which is soluble in a weakly acidic or neutral medium and which, in Step b), is precipitated onto the substrate by adding acid and/or metal salts, such as an alkali metal salt, alkaline earth metal salt, aluminium salt or transition metal salt, especially NH₄⁺, ⁺NR³¹R³²R³³R³⁴, wherein R³¹, R³², R³³ and R³⁴ are as defined hereinbelow, Na, K, Mg, Ca, Sr, Ba, Mn, Cu, Ni, Cd, Co, Cr, Zn, Al or Fe. Special preference is given to salts of calcium and aluminium.

Examples of such colorants are described, for example, in WO2002/085988 A1 and W02003/066938 A2.

Examples of pigments (and also substituted derivatives thereof) that may be used as starting compounds and that fall within the above-mentioned pigment classes are described in W. Herbst, K. Hunger, Industrielle Organische Pigmente, 2nd completely revised edition, VCH 1995: 1-aminoanthraquinone pigments: p. 503-511; anthraquinone pigments: p. 504-506, 513-521 and 521-530; anthrapyrimidine: p. 513-415; azo pigments: p. 219-324 and 380-398; azomethine pigments: p. 402 - 411; quinacridone pigments: p. 462-481; quinacridone quinone pigments: p. 467-468; quinophthalone pigments: p. 567-570; diketopyrrolopyrrole pigments: p. 570-574; dioxazine pigments: p. 531-538; flavanthrone pigments: p. 517-519, 521; indanthrone pigments: p. 515-517; isoindoline pigments: p. 413-429; isoindolinone pigments: p. 413-429; isoviolanthrone pigments: p. 528-530; perinone pigments: p. 482-492; perylene pigments: p. 482-496; phthalocyanine pigments: p. 431-460; pyranthrone pigments: p. 522-526; thioindigo pigments (indigo pigments): p. 497-500, it also being possible to use mixtures of such pigments, including solid solutions.

The present invention relates also to a method in which SiO₂ and a pigment are applied by precipitation at the same time as a colorant.

In a preferred embodiment, the colorant, for example tetrakis(sulfon-4-amidosalicylic acid) copper phthalocyanine, is dissolved at room temperature in water, adding sodium silicate (soda waterglass) and sodium hydroxide solution until a pH of from 8 to 12 has been reached. The substrate particles, for example aluminium flakes (Eckart Aloxal 3010) are stirred into the resulting solution at from 0 to 30°C, preferably at room temperature, and mixed thoroughly. The alkaline dispersion is acidified to pH 3.5, whereupon the colour of the solution changes markedly. The substrate particles are isolated in customary manner, for example by filtering off, washing and drying. The product, which has a metallic blue shimmer, obtained in the case of tetrakis(sulfon-4-amidosalicylic acid) copper phthalocyanine can be incorporated into plastics and surface-coating compositions without further after-treatment and yields a blue metallic effect.

According to the method described hereinbefore, in addition to the deposition of SiO₂ and pigment or SiO₂, colorant and pigment, it is also possible for dispersed pigment particles and SiO₂ to be deposited onto substrates.

Known chromophores having the basic structure A(H)ₓ are for example, 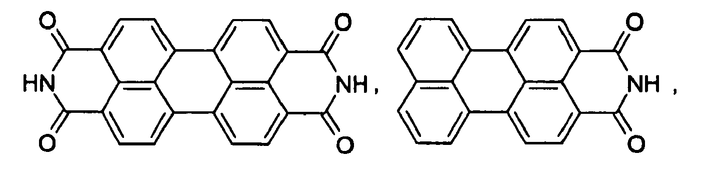 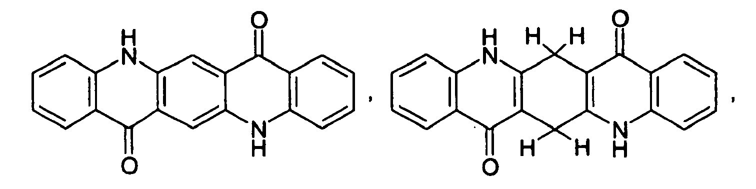 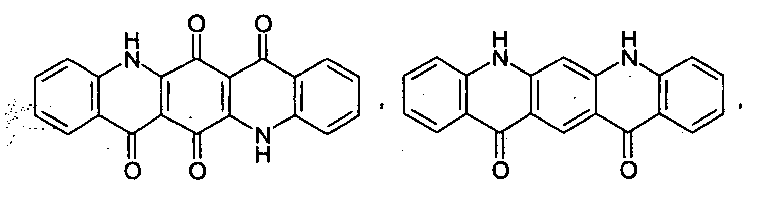 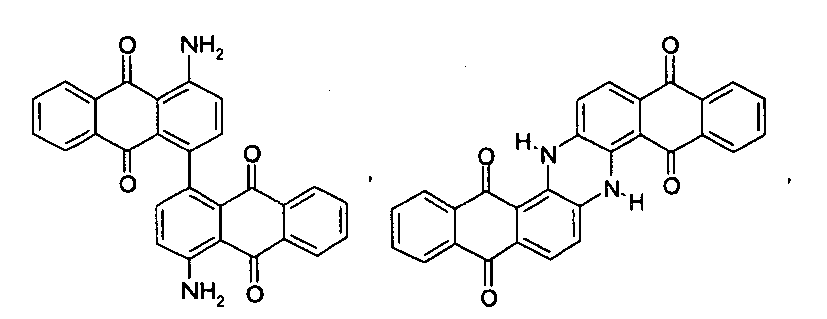 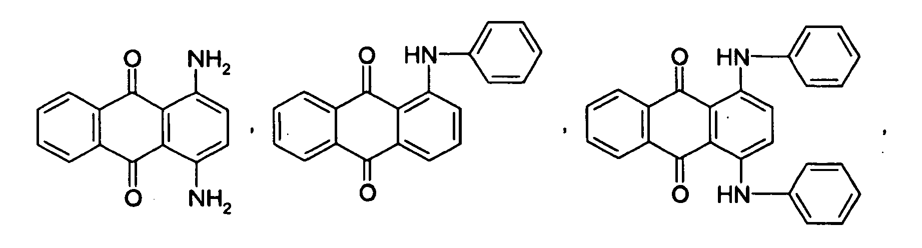 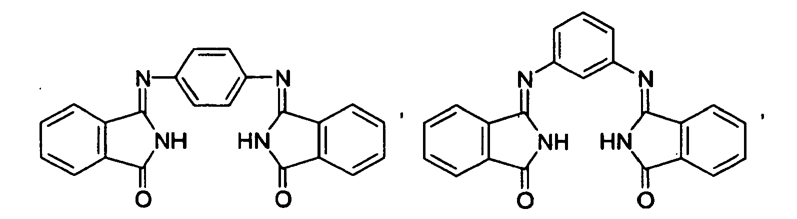 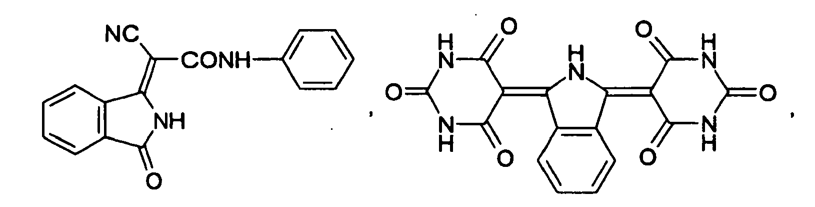 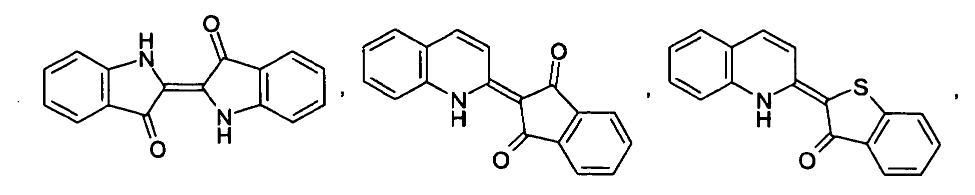 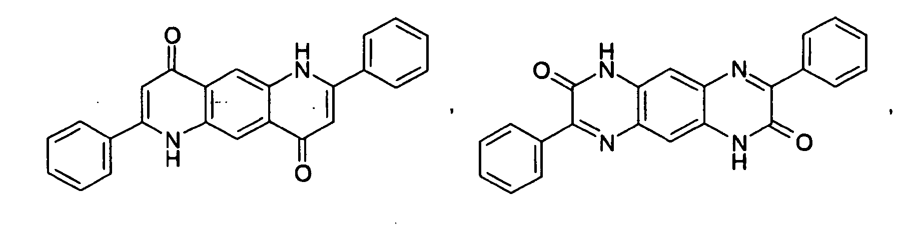 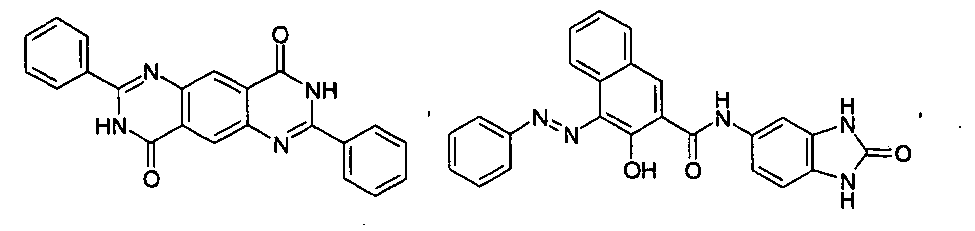 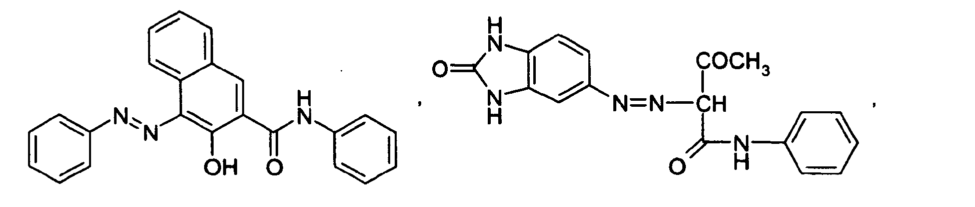 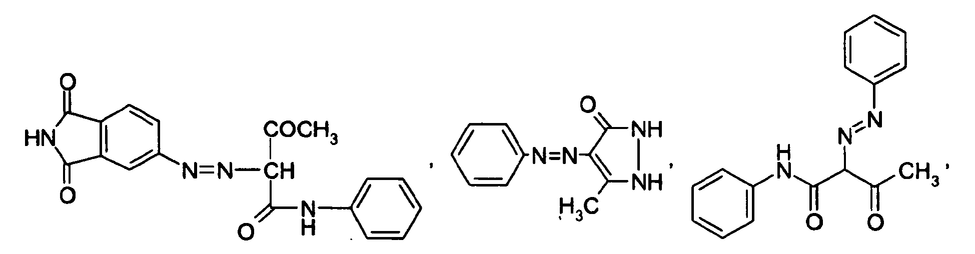 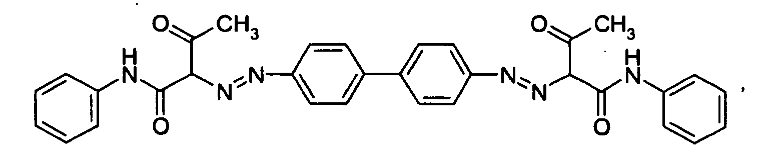 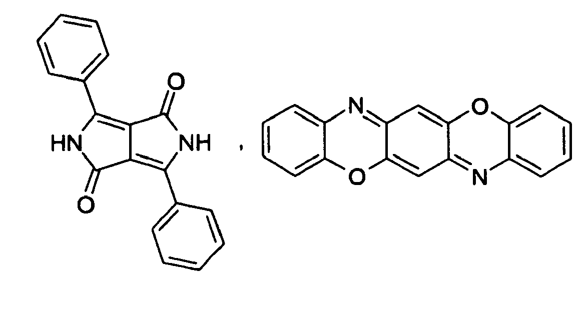 or 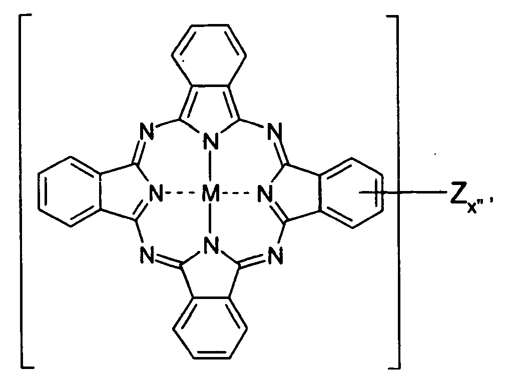 wherein, for example, Z is 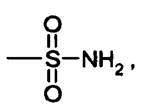 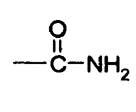 or -CH₂-NH₂, and x" is a number from 1 to 16, especially from 1 to 4;
and also, in each case, all known derivatives thereof.

Worthy of special mention are those soluble chromophores wherein the pigment of formula A(H)ₓ is Colour Index Pigment Yellow 13, Pigment Yellow 73, Pigment Yellow 74, Pigment Yellow 83, Pigment Yellow 93, Pigment Yellow 94, Pigment Yellow 95, Pigment Yellow 109, Pigment Yellow 110, Pigment Yellow 120, Pigment Yellow 128, Pigment Yellow 139, Pigment Yellow 151, Pigment Yellow 154, Pigment Yellow 175, Pigment Yellow 180, Pigment Yellow 181, Pigment Yellow 185, Pigment Yellow 194, Pigment Orange 31, Pigment Orange 71, Pigment Orange 73, Pigment Red 122, Pigment Red 144, Pigment Red 166, Pigment Red 184, Pigment Red 185, Pigment Red 202, Pigment Red 214, Pigment Red 220, Pigment Red 221, Pigment Red 222, Pigment Red 242, Pigment Red 248, Pigment Red 254, Pigment Red 255, Pigment Red 262, Pigment Red 264, Pigment Brown 23, Pigment Brown 41, Pigment Brown 42, Pigment Blue 25, Pigment Blue 26, Pigment Blue 60, Pigment Blue 64, Pigment Violet 19, Pigment Violet 29, Pigment Violet 32, Pigment Violet 37, 3,6-di(4'-cyano-phenyl)-2,5-dihydro-pyrrolo[3,4-c]pyrrole-1,4-dione, 3,6-di(3,4-dichloro-phenyl)-2,5-dihydro-pyrrolo-[3,4-c]pyrrole-1,4-dione or 3-phenyl-6-(4'-tert-butyl-phenyl)-2,5-dihydro-pyrrolo[3,4-c]pyrrole-1,4-dione. Further examples are described by Willy Herbst and Klaus Hunger in "Industrial Organic Pigments" (ISBN 3-527-28161-4, VCH / Weinheim 1993). In general, those soluble pigment precursors do not have deprotonatable carboxylic acid or sulfonic acid groups.

Alkyl or alkylene may be straight-chained, branched, monocylic or polycyclic. C₁-C₁₂Alkyl is accordingly, for example, methyl, ethyl, n-propyl, isopropyl, n-butyl, sec-butyl, isobutyl, tert-butyl, cyclobutyl, n-pentyl, 2-pentyl, 3-pentyl, 2,2-dimethylpropyl, cyclopentyl, cyclohexyl, n-hexyl, n-octyl, 1,1,3,3-tetramethylbutyl, 2-ethylhexyl, nonyl, trimethylcyclohexyl, decyl, menthyl, thujyl, bornyl, 1-adamantyl, 2-adamantyl or dodecyl.

When C₂-C₁₂alkyl is mono- or poly-unsaturated, it is C₂-C₁₂alkenyl, C₂-C₁₂alkynyl, C₂-C₁₂alkapolyenyl or C₂-C₁₂alkapolyynyl, it being possible for two or more double bonds to be, where appropriate, isolated or conjugated, such as, for example, vinyl, allyl, 2-propen-2-yl, 2-buten-1-yl, 3-buten-1-yl, 1,3-butadien-2-yl, 2-cyclobuten-1-yl, 2-penten-1-yl, 3-penten-2-yl, 2-methyl-1-buten-3-yl, 2-methyl-3-buten-2-yl, 3-methyl-2-buten-1-yl, 1,4-pentadien-3-yl, 2-cyclopenten-1-yl, 2-cyclohexen-1-yl, 3-cyclohexen-1-yl, 2,4-cyclohexadien-1-yl, 1-p-menthen-8-yl, 4(10)-thujen-10-yl, 2-norbornen-1-yl, 2,5-norbornadien-1-yl, 7,7-dimethyl-2,4-norcaradien-3-yl and the various isomers of hexenyl, octenyl, nonenyl, decenyl and dodecenyl. C₂-C₄Alkylene is, for example, 1,2-ethylene, 1,2-propylene, 1,3-propylene, 1,2-butylene, 1,3-butylene, 2,3-butylene, 1,4-butylene and 2-methyl-1,2-propylene. C₅-C₁₂Alkylene is, for example, an isomer of pentylene, hexylene, octylene, decylene or dodecylene.

C₁-C₁₂Alkoxy is O-C₁-C₁₂alkyl, preferably O-C₁-C₄alkyl.

C₆-C₁₂Aryloxy is O-C₆-C₁₂aryl, for example phenoxy or naphthyloxy, preferably phenoxy. C₁-C₁₂Alkylthio is S-C₁-C₁₂alkyl, preferably S-C₁-C₄alkyl.

C₆-C₁₂Arylthio is S-C₆-C₁₂aryl, for example phenylthio or naphthylthio, preferably phenylthio. C₂-C₂₄Dialkylamino is N(alkyl₁)(alkyl₂), the sum of the carbon atoms in the two groups alkyl₁ and alkyl₂ being from 2 to 24, preferably N(C₁-C₄alkyl)-C₁-C₄alkyl.

C₇-C₂₄Alkylarylamino is N(alkyl₁)(aryl₂), the sum of the carbon atoms in the two groups alkyl₁ and aryl₂ being from 7 to 24, for example methylphenylamino, ethylnaphthylamino or butylphenanthrylamino, preferably methylphenylamino or ethylphenylamino. C₁₂-C₂₄Diarylamino is N(aryl₁)(aryl₂), the sum of the carbon atoms in the two groups aryl₁ and aryl₂ being from 12 to 24, for example diphenylamino or phenylnaphthylamino, preferably diphenylamino.

Halogen is chlorine, bromine, fluorine or iodine, preferably fluorine or chlorine, especially chlorine.

The coloured (flake-like) carrier particles according to the invention can be used wherever pigments and effect pigments are normally used. An overview of various possibilities for using effect pigments and the compositions used therein is included, for example, in WO2003/068868 A2, WO04/020530 A1, PCT/EP03/68868, WO04/035684 A2, WO04/020530 A1 and WO04/035911 A2.

The present invention accordingly relates also to the use of the coloured carrier particles for colouring textiles, coating compositions, printing inks, plastics, glass, ceramic products and cosmetic preparations, and also in ink jet printing and to formulations (preparations) for the above-mentioned applications. All customary printing processes can be employed, for example offset printing, intaglio printing, bronzing, flexographic printing.

The pigments of the present invention can be used in admixture with filler pigments, colored and black organic and inorganic pigments, colored and black luster pigments based on, for example, metal oxide coated mica, holographic pigments, liquid crystal polymers (LCPs), or conventional metal pigments.

The concentration of the pigments in the system in which it is to used dependent on the specific application, but is generally between 0.01 and 50 % by weight, preferably between 0.1 and 5 % by weight, based on the overall solids content of the system.

Plastics comprising the pigment of the invention in amounts of 0.1 to 50 % by weight, in particular 0.5 to 7 % by weight.

In the coating sector, the pigments of the invention are employed in amounts of 0.5 to 10 % by weight.

In the pigmentation of binder systems, for example for paints and printing inks for intaglio, offset or screen printing, the pigment is incorporated into the printing ink in amounts of 2 to 50 % by weight, preferably 5 to 30 % by weight and in particular 8 to 15 % by weight.

The pigments according to the invention are also suitable for making-up the lips or the skin and for colouring the hair or the nails. The invention accordingly relates also to a cosmetic preparation or formulation comprising from 0.0001 to 90 % by weight of a pigment, especially an pigment, according to the invention and from 10 to 99.9999 % of a cosmetically suitable carrier material, based on the total weight of the cosmetic preparation or formulation. Such cosmetic preparations or formulations are, for example, lipsticks, blushers, foundations, nail varnishes and hair shampoos.

The pigments may be used singly or in the form of mixtures. It is, in addition, possible to use pigments according to the invention together with other pigments and/or colorants, for example in combinations as described hereinbefore or as known in cosmetic preparations.

The cosmetic preparations and formulations according to the invention preferably contain the pigment according to the invention in an amount from 0.005 to 50 % by weight, based on the total weight of the preparation. Suitable carrier materials for the cosmetic preparations and formulations according to the invention include the customary materials used in such compositions (see, for example, WO2003/076520 A2).

The cosmetic preparations and formulations according to the invention may be in the form of, for example, sticks, ointments, creams, emulsions, suspensions, dispersions, powders or solutions. They are, for example, lipsticks, mascara preparations, blushers, eye-shadows, foundations, eyeliners, powder or nail varnishes.

If the preparations are in the form of sticks, for example lipsticks, eye-shadows, blushers or foundations, the preparations consist for a considerable part of fatty components, which may consist of one or more waxes, for example ozokerite, lanolin, lanolin alcohol, hydrogenated lanolin, acetylated lanolin, lanolin wax, beeswax, candelilla wax, microcrystalline wax, carnauba wax, cetyl alcohol, stearyl alcohol, cocoa butter, lanolin fatty acids, petrolatum, petroleum jelly, mono-, di- or tri-glycerides or fatty esters thereof that are solid at 25°C, silicone waxes, such as methyloctadecane-oxypolysiloxane and poly(dimethylsiloxy)stearoxysiloxane, stearic acid monoethanolamine, colophane and derivatives thereof, such as glycol abietates and glycerol abietates, hydrogenated oils that are solid at 25°C, sugar glycerides and oleates, myristates, lanolates, stearates and dihydroxystearates of calcium, magnesium, zirconium and aluminium.

The fatty component may also consist of a mixture of at least one wax and at least one oil, in which case the following oils, for example, are suitable: paraffin oil, purcelline oil, perhydrosqualene, sweet almond oil, avocado oil, calophyllum oil, castor oil, sesame oil, jojoba oil, mineral oils having a boiling point of about from 310 to 410°C, silicone oils, such as dimethylpolysiloxane, linoleyl alcohol, linolenyl alcohol, oleyl alcohol, cereal grain oils, such as wheatgerm oil, isopropyl lanolate, isopropyl palmitate, isopropyl myristate, butyl myristate, cetyl myristate, hexadecyl stearate, butyl stearate, decyl oleate, acetyl glycerides, octanoates and decanoates of alcohols and polyalcohols, for example of glycol and glycerol, ricinoleates of alcohols and polyalcohols, for example of cetyl alcohol, isostearyl alcohol, isocetyl lanolate, isopropyl adipate, hexyl laurate and octyl dodecanol.

The fatty components in such preparations in the form of sticks may generally constitute up to 99.91 % by weight of the total weight of the preparation.

The cosmetic preparations and formulations according to the invention may additionally comprise further constituents, such as, for example, glycols, polyethylene glycols, polypropylene glycols, monoalkanolamides, non-coloured polymeric, inorganic or organic fillers, preservatives, UV filters or other adjuvants and additives customary in cosmetics, for example a natural or synthetic or partially synthetic di- or tri-glyceride, a mineral oil, a silicone oil, a wax, a fatty alcohol, a Guerbet alcohol or ester thereof, a lipophilic functional cosmetic active ingredient, including sun-protection filters, or a mixture of such substances.

A lipophilic functional cosmetic active ingredient suitable for skin cosmetics, an active ingredient composition or an active ingredient extract is an ingredient or a mixture of ingredients that is approved for dermal or topical application.

The Examples that follow illustrate the present invention, without limiting the scope thereof. Percentages and parts are always percentages by weight and parts by weight, respectively, unless otherwise specified.

### Examples

### Example 1 (example 1 does not represent the invention)

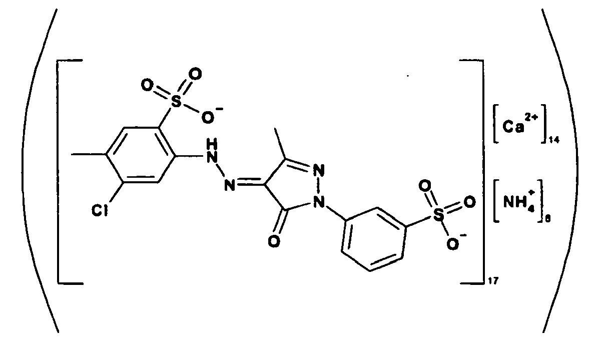

0.40 g of the disulfonic acid of Pigment Yellow 191 is introduced into a 600 ml glass beaker with 100.0 g of deionised water and 1.5 g of sodium silicate, heated to 80°C and stirred. At 70°C, the orange solution is filtered and introduced into a 600 ml glass beaker and, whilst stirring at room temperature, 1.0 g of Aloxal® 3010 (Eckart GmbH & Co. KG) is introduced and a homogeneous suspension is produced. Using 9.0 g of 2 % hydrochloric acid solution, the pH value is quickly brought to 6.0, the dye remaining in solution. Then, 0.11 g of calcium chloride and 0.034 g of ammonium acetate, dissolved in 20 g of water, are slowly added dropwise, whereupon the pH value is 6.4. Using 0.7 g of 2 % hydrochloric acid solution, the pH value is slowly lowered to 4.4, whereupon the yellow pigment is deposited onto the aluminium flakes in the form of an NH₄/Ca lake. The mixture is stirred vigorously for 25 min., heated to 70°C and, at that temperature, stirred for a further hour. With the heating stopped, the mixture is stirred for one hour more and is allowed to cool to room temperature. The pH value is then 4.6.

After filtration and rinsing with 20 g of water, the filter cake is dried first for 48 hours at room temperature and then for 8 hours at 60°C and 100 hPa in a vacuum drying cabinet. A yellow pigment powder having a metal effect is obtained.

| **Example** | **Dye** | **% dye** | **% aluminium flakes** | **Laking** |
|---|---|---|---|---|
| 1 | Pigment Yellow 191 (di-SO₃H) | 40 | 100 | NH₄⁺/Ca²⁺ |

### Example 2

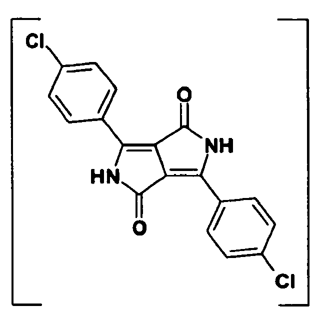

0.60 g of Pigment Red 254 is introduced into a 400 ml glass beaker in 100.00 g of deionised water, 20.00 g of methanol, 2.00 g of sodium silicate solution and 100.00 g of glass beads (diameter: 2 mm) and dispersed for 2 hours. The red suspension is filtered over a glass filter plate and the glass beads are rinsed with 50 g of deionised water. The red suspension is introduced into a 600 ml glass beaker and stirred at room temperature, and 4.00 g of sodium silicate solution are introduced. The pH value is slowly brought to 10.5 by adding 10.0 g of 4 % hydrochloric acid solution. Cleaned aluminium flakes (Aloxal® 3010) are then introduced. Whilst stirring vigorously, the pH value is adjusted to 5.8 over a period of 1 minute, using 8.90 g of 4 % hydrochloric acid solution.

The mixture is then heated at 50°C for 1 hour, with stirring. The red pigment is deposited together with sodium silicate in the form of a gel onto the aluminium flakes. The pH value is 6.6 and is slowly brought to 3.1, using 0.40 g of 4 % hydrochloric acid solution. Then, 5 % sodium stearate solution (0.07 g of sodium stearate dissolved at 50°C in 10 g of water) is slowly added dropwise, whereupon the pH value is 3.7; the mixture is stirred vigorously for 10 minutes, then stirred for a further 2 hours with the heating stopped, and cooled to room temperature.

Filtration is carried out using a suction filter, subsequently washing with 200.0 g of deionised water. The moist filter cake is dried first for 16 hours in air at room temperature and then for 4 hours at 60°C and 100 hPa in a vacuum drying cabinet, resulting in a red metal effect pigment.

### Examples 3 - 8

Coating is performed analogously to Example 2. The pigments used in Examples 2 to 8 and the amounts of pigment and aluminium flakes are given in the following Table.

| **Example** | **Pigment** | **% pigment** | **% aluminium flakes** | **Coating** |
|---|---|---|---|---|
| 2 | Pigment Red 254 | 75 | 100 | 5 % sodium stearate |
| 3 | Pigment Red 179 (Irgazin® Red 2273) | 100 | 100 | 5 % sodium stearate |
| 4 | Pigment Red 179 (Irgazin® Maroon 3379) | 100 | 100 | 5 % sodium stearate |
| 5 | Pigment Red 264 | 100 | 100 | 5 % sodium stearate |
| 6 | Pigment Yellow 109 | 100 | 100 | 5 % sodium stearate |
| 7 | Pigment Yellow 110 | 100 | 100 | 5 % sodium stearate |
| 8 | Pigment Blue 15:3; copper phthalocyanine (β) | 80 | 100 | 5 % sodium stearate |

## Claims

1. A method of producing coloured carrier particle, which comprises
a) dispersing a pigment in aqueous solution,
b) adding soda waterglass,
c) precipitating SiO₂ and the pigment onto the carrier particles by lowering the pH value.

2. Coloured carrier particles obtainable by the method according to claim 1.

3. Use of the coloured carrier particles according to claim 2 in colouring textiles, surface-coating compositions, printing inks, plastics, glass, ceramic products or cosmetic preparations or in ink jet printing.

## Patentansprüche

1. Verfahren zur Herstellung von gefärbten Trägerpartikeln, umfassend
a) das Dispergieren eines Pigmentes in wässriger Lösung,
b) die Zugabe von Natronwasserglas,
c) das Ausfällen von SiO₂ und des Pigments auf die Trägerpartikel durch Erniedrigung des pH-Werts.

2. Gefärbte Trägerpartikel, erhältlich nach dem Verfahren gemäss Anspruch 1.

3. Verwendung der gefärbten Trägerpartikel nach Anspruch 2 zur Einfärbung von Textilien, Lacken, Druckfarben, Kunststoffen, Glas, keramischen Produkten oder kosmetischen Präparaten oder im Tintenstrahldruck.

## Revendications

1. Procédé pour la fabrication de particules de support colorées, qui comprend
a) disperser un pigment dans solution aqueux,
b) ajouter du verre soluble de soude,
c) précipiter du SiO₂ et le pigment sur les particules de support en abaissant la valeur pH.

2. Particules de support colorées procurables selon le procédé de la revendication 1.

3. Utilisation des particules de support colorées selon la revendication 2 pour la coloration de textiles, de lacques, d'encres d'imprimerie, de matières plastiques, de verre, de produits céramiques ou de préparations cosmétiques, ou pour l'impression au jet d'encre.
